(19) Europäisches Patentamt
European Patent Office
Office européen des brevets

(11) **EP 4 640 133 A1**

(12) **EUROPEAN PATENT APPLICATION**

(43) Date of publication:
**29.10.2025 Bulletin 2025/44**

(21) Application number: **25171577.7**

(22) Date of filing: **22.04.2025**

(51) International Patent Classification (IPC):
***A61B 3/024*** *(2006.01)*

(52) Cooperative Patent Classification (CPC):
**A61B 3/024**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **23.04.2024 IT 202400009325**

(71) Applicant: **New Digital Consulting S.r.l. - Società Benefit**
**84134 Salerno (IT)**

(72) Inventor: **SANGIUOLO, Raffaele**
**84134 Salerno (IT)**

(74) Representative: **Cutropia, Gianluigi**
**Ing. Claudio Baldi s.r.l.**
**Viale Cavallotti, 13**
**60035 Jesi (AN) (IT)**

(54) **DIGITAL TECHNOLOGY SYSTEM FOR ASSESSMENT OF THE MACULAR FUNCTION COEFFICIENT**

(57)     A system (100) for assessing a macular function percentage comprises: a visor (1) with internal display (10), a patient interface (2), a user interface (3) with external display (30), a power supply (5), and a control unit (4) configured to display a background (S) and luminance targets (M) on the internal display; the patient interface (2) is configured to be operated by the patient to select each luminance target seen by the patient on the internal display (10); the control unit (4) is configured to perform a test comprising a plurality of steps; and the external display (30) is configured to display a result of the test performed.

FIG. 2

EP 4 640 133 A1

**Description**

The present invention relates to a visor device and to a method based on digital technology for assessing a macular function percentage coefficient.

A numerical assessment of the macular function expressed with a percentage coefficient is of utmost importance in the field of maculopathies.

In particular, such an assessment system is most useful for the study of senile and juvenile macular degeneration (such as Best's disease or Stargardt's disease), macular edema, myopic retinopathy, diabetic retinopathy, and macular holes.

In addition, such an assessment system can be useful in follow-ups that are performed following retinal therapies, i.e. after intravitreal injections to treat the exudative form of age-related macular degeneration (AMD).

Moreover, such an evaluation system is important in guiding therapeutic choices in the period following surgical operations and in evaluating the results of the operations; moreover, it can play an important role in the realization of rehabilitation programs for visually impaired patients.

In order to understand the importance of such a type of assessment, it is enough to consider that, in Italy, more than one million people suffer from a form of maculopathy and that 63,000 new cases are diagnosed each year. These numbers are inevitably destined to increase due to the progressive aging of the population, as well as to the increase of risk factors, such as diabetes.

Currently, the assessment of macular function is performed with retinal microperimetry, which is a diagnostic examination used to study the central retinal sensitivity, correlating the morphological aspect with the functional aspect. More specifically, retinal microperimetry is used to evaluate the patient's ability to see luminous stimuli of varying intensity at different locations in the retinal region under examination. The information obtained from such a test provides a better understanding of the macular function by creating a sensitivity map of the central retinal region.

Currently, no digital tool is available to perform a numerical assessment of the macular function, which is valuable both for the screening of maculopathy and for the monitoring of its evolution, in order to optimize the therapeutic systems.

Similarly, a digital technology system capable of expressing a percentage coefficient of the macular function through a rapid procedure to obtain numerical results is not available so far.

US2019150727A1 describes a system for assessment of a visual field of a user. A target is displayed on a display. After establishing that the user has seen the target and after determining the position of the user's eyes, a test target is displayed on the display at a location that corresponds to a location of the user's field of vision. The detection of the test target is determined based on an input of the user that is acquired while the user is looking at the display.

The purpose of the present invention is to eliminate the drawbacks of the prior art by providing a system for assessing the macular function that is efficient, rapid, and reliable.

Another purpose is to provide such a system for the assessment of the macular function that is versatile, practical and easy to implement.

These purposes are achieved in accordance with the invention with the features of the attached independent claims.

Advantageous embodiments of the invention appear from the dependent claims.

The system for the assessment of the macular function according to the invention is defined in claim 1.

The digital system according to the invention has a number of advantages over microperimetry:

- the test takes place in virtual reality, with no interference from environmental factors;
- the test is performed in a highly immersive environment;
- at the end of the test, the numerical value of the macular function percentage coefficient is expressed and automatically processed by a software installed in the digital visor, so that the results can be easily compared over time;
- the test has a high speed of execution (average 3 to 5 minutes)
- the test gives the possibility of connecting several visor devices in order to facilitate communication between different eye care centers; this aspect is particularly interesting in the case of polycentric studies.

Further features of the invention will appear clearer from the detailed description that follows, referring to a purely illustrative and therefore non-limiting embodiment, illustrated in the accompanying drawings, wherein:

Fig. 1 is a perspective view, illustrating the system for the assessment of macular function according to the invention;

Fig. 2 is a block diagram of the system in Fig. 1;

Fig. 3 is a view of a screenshot of the internal display of the visor of the system of Fig. 1;

Fig. 4 is a view illustrating a grid used for positioning the targets in the screenshot of the visor of the Fig. 1;

Fig. 5 is a graph illustrating a timing of the luminance targets;

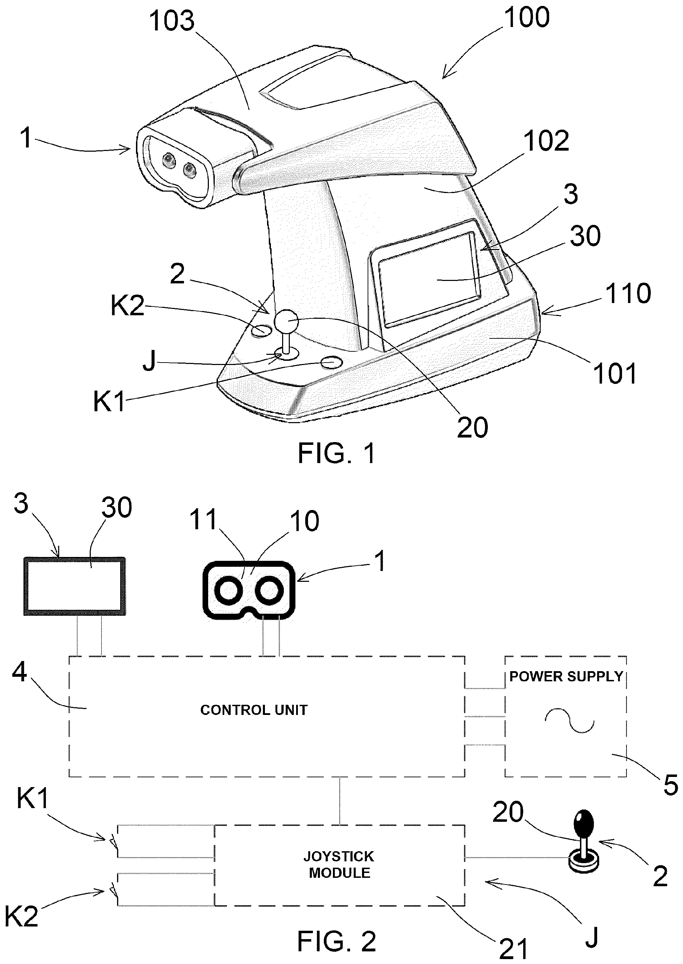

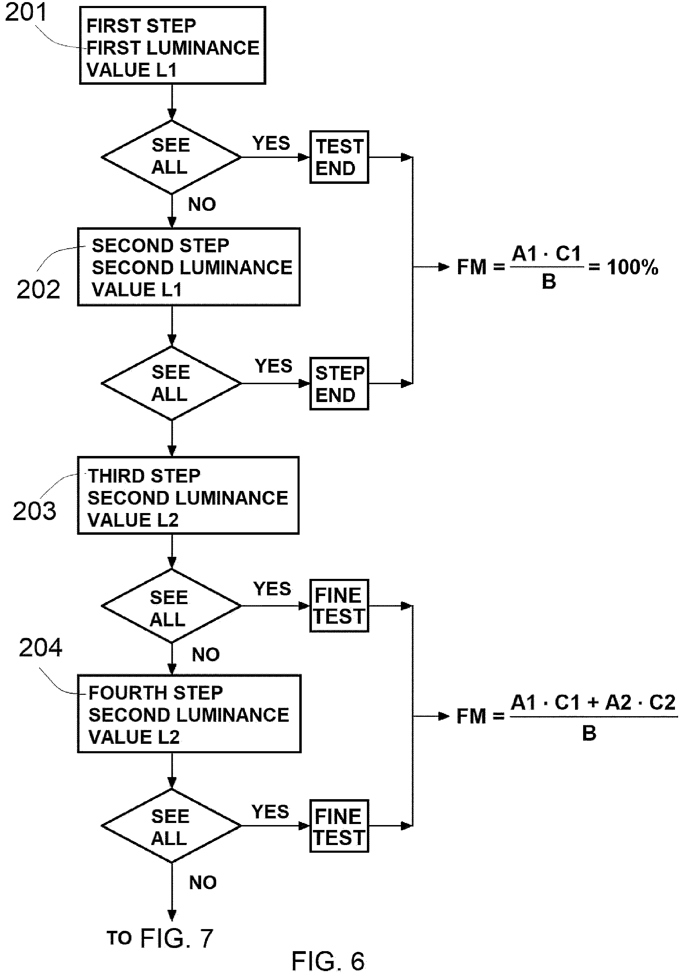

he Fig. 6 is a first part of a flowchart, illustrating the procedure for calculating the macular function with the system in Fig. 1;

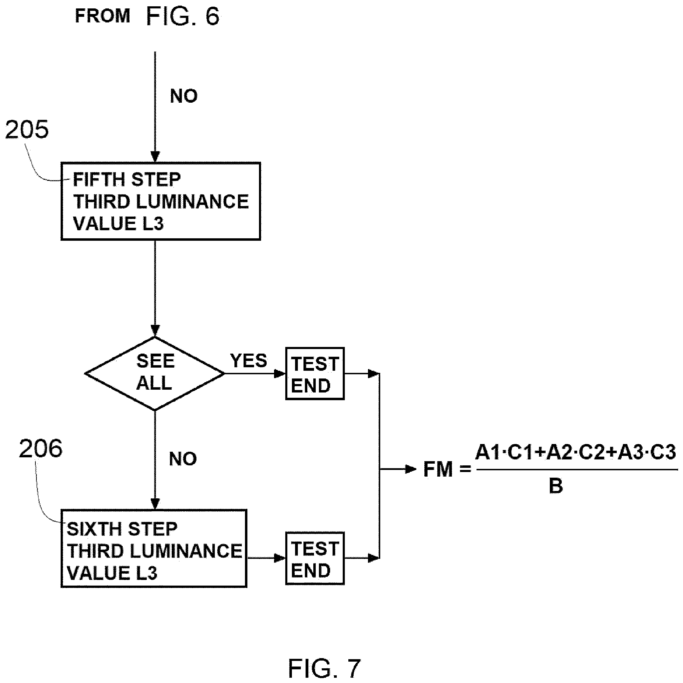

Fig. 7 is a second part of the flowchart of Fig. 6; and

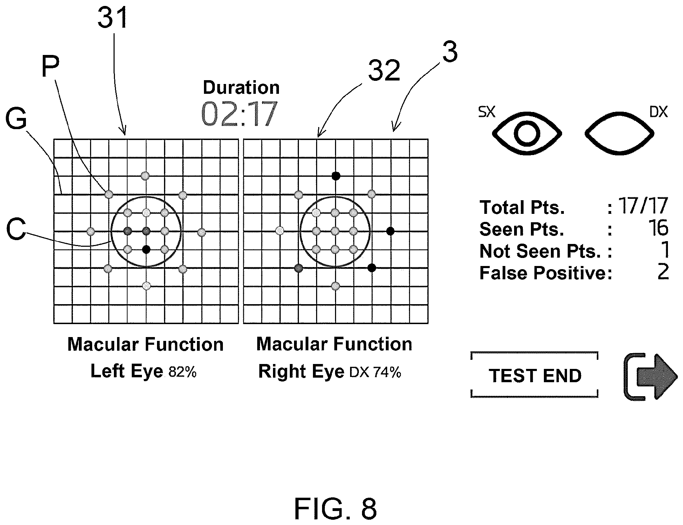

Fig. 8 is an image as seen in the external display of the system at the end of the test;

With reference to the Figures, the system for assessing the macular function according to the invention, which is comprehensively referred to as reference number 100, is described.

Now with reference to Fig. 1, the system (100) comprises a boxed body (110).

The body (110) comprises:

- a base (101) suitable for being placed on a horizontal plane,
- an upright (102) that rises from the base, and
- an upper part (103) arranged on the upright (102).

A digital visor (1) is mounted in the upper part (103) of the body, suitable for being used by the patient. For this purpose, the visor (1) protrudes anteriorly from the upright (102), so that the patient's forehead and face can be rested on the visor.

Referring to Fig. 2, the visor (1) comprises an internal display (10) and optical detection means (11) such as optical sensors and/or cameras capable of detecting the patient's pupils. The visor (1) may also comprise biconvex lenses, illuminators, and an adjustment system of the interpupillary distance.

Going back to Fig. 1, a patient interface (2) is provided in the base (101). The patient interface (2) comprises a joystick (J) and/or buttons (K1, K2).

The joystick (J) has a lever (20) that protrudes superiorly from the base (101) in front of the upright (102). A right button (K1) and a left button (K2) are arranged on the base (101), to the right and left of the lever (20) of the joystick, respectively. The joystick (J) and the buttons (K1, K2) are suitable for being used by the patient while performing the test.

A user interface (3) is provided in the upright (102). The user interface (3) comprises an external display (30) arranged, for example, in a right side of the upright (102). The external display (30) is to be viewed by a user who should be an ophthalmologist or a trained technician. The external display (30) is a touch screen display.

With reference to Fig. 2, a control unit (4), a power supply (5) and a joystick module (21) are arranged in the shell (110).

The control unit (4) is operatively connected to the visor (1), to the external display (30), to the power supply (5) and to the joystick module (21). The joystick module (21) is operatively connected to the lever (20) of the joystick and to the buttons (K1, K2).

The joystick module (21) is configured to control the commands given by the lever (20) of the joystick and by the buttons (K1, K2). Obviously, the joystick module (21) can be integrated into the control unit (4).

The power supply (5) is of medical type and can be arranged inside the body (110) or outside the body (110) or can be integrated in the control unit (4).

The control unit (4) comprises a processing unit, a graphic board, memories and connections such as video, Wi-Fi, USB, LAN inputs and outputs and the like.

When the user brings his or her eyes closer to the visor (1), the optical detection means (11) of the visor frame the pupils of the user's eyes and sends the pupil images to the control unit (4). The control unit (4) has an eye tracking software that detects a correct fixation position of the pupil that will be visible to the operator at all times during the test via the external display (30).

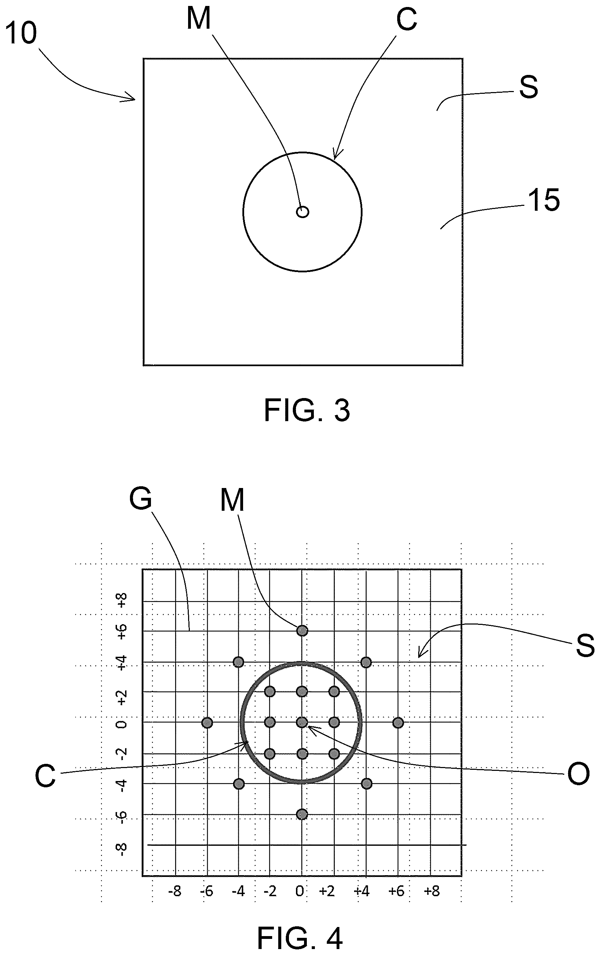

Referring to Fig. 3, the control unit (4) has a software configured in such a way that the internal display (10) of the visor can display a screenshot (15) with a background (S) on which luminance targets (M) in the form of luminous points appear, one at a time.

The background (S) and the luminance targets (M) generate a virtual reality scenario with specially designed brightness characteristics in order to create a proper contrast ratio between the background (S) and the luminance targets (M), given by the luminance of the targets (M) as explained below. By way of example, the targets (M) are whiter and the background (S) is preferably blue, green or brown.

The luminance of the targets (M) is set by a software program installed in the visor (1).

The luminance targets (M) appear on the background (S) at predetermined locations, with a random order and randomized time intervals.

A circle (C) that identifies a central fixation zone also appears in central position in the screen (15) of the internal display (10).

Referring to Fig. 4, the positions at which the luminance targets (M) appear are described. In order to identify these positions, a grid (G) is disposed in the background (S) to define the area in which the luminance targets (M) are presented. In the area defined by the grid (G), the distances from a central fixation point (O) (origin) are measured in degrees.

The extent of the grid (G) corresponds to the entire macular function area. The extent of the grid (G) is 12° vertically (6° in the upper quadrants and 6° in the lower quadrants relative to the central fixation point (O)) and 12° horizontally (6° in the nasal quadrant and 6° in the temporal quadrant relative to the central fixation point (O)).

It should be noted that, according to the division in degrees made by the grid (G), each luminance target (M) has a diameter of 0.6°.

A circle (C) is drawn on the grid (G) to define a central fixation zone. The circle (C) has its center at the central fixation point (O) and a radius of 4°. The central fixation circle (C) must not interfere with any presentation area of the luminance targets. It was chosen to define a fixation zone with a circle, and not with a single one central point, because patients with macular pathology are not always able to perceive a single central fixation point.

During a first step of the test, a plurality of luminance targets (M) equal to a predetermined number N are presented progressively, one by one, in the internal display (10).

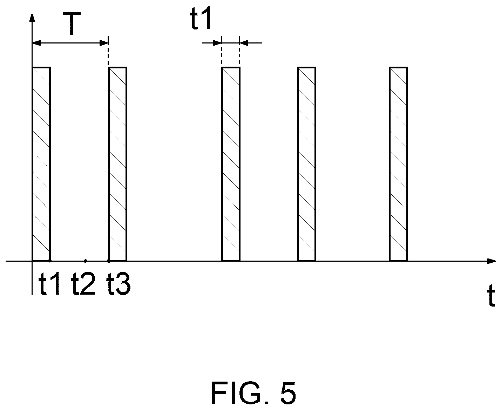

With reference to Fig. 5, a timing of the luminance targets (M) that appear in the background (S) of the internal display is illustrated.

Each target (M) remains visible in the internal display for a display time (t1) comprised between 0.2 - 0.5 s. Thus each luminance target can be obtained as a pulse of duration t1.

If the patient sees the luminance target, he/she reports it by using the patient interface (2), for example, by indifferently pressing the right button (K1) or the left button (K2). The patient has a reaction time (t2) of 1 s from the moment in which the target is presented to give a feedback with the patient interface.

If the patient reports seeing the target outside the reaction time (t2), a false positive is reported.

A randomized presentation time (t3) passes between the appearance of a target and the following target, said randomized presentation time being obviously greater than the reaction time (t2). Therefore, the presentation time (t3) can be chosen randomly in the range of 1 to 1.5 s.

This prevents the user from automatically reporting the presence of the target at regular intervals, without actually seeing it, by randomly entering the reaction time (t2).

Therefore, the presentation of the luminance targets can be represented as a train of pulses having a fixed duration t1 and a period T = t3 that is randomly randomized in a time interval.

It was chosen to use 17 luminance targets positioned as in the graph of Fig. 4.

Nine luminance targets are positioned according to a 3x3 array, inside the central fixation circle (C), in which the central luminance target is located at the central fixation point (O) and the other luminance targets are spaced by 2°. Eight luminance targets are positioned outside the central fixation circle (C) along eight equally spaced points of a circumference of radius 6°.

With reference to Figs. 6 and 7, the test is described.

The test begins with an initial step (201) in which the N luminance targets (M) appear progressively, one by one, on the internal display (10) and the patient reports their vision with the patient interface (2).

In the first stage, the targets (M) have a first luminance (L1), for example 1.62 lux - 2.1db.

If the patient sees all the targets correctly, the test ends with a maximum macular function (FM) coefficient of 100%.

If the patient does not see some of the targets, a second step (202) is performed, in which only the targets not seen in the first step are presented for a second time at the same luminance (L1) as in the first step. If the patient correctly sees all the targets in the second step, the test ends with a maximum macular function coefficient (FM) of 100%.

If the patient does not see some of the targets in the second step, a third step (203) is performed, in which only the luminance targets (M) not seen in the second step appear progressively, one by one, on the internal display (10) and the patient reports their vision with the patient interface (2). In the third step, the targets (M) have a second luminance (L2), e.g. 1.97 lux - 2.9db, which is higher than the first luminance (L1).

If the patient does not see some of the target presented in the third step, a fourth step (204) is performed, in which the targets not seen in the third step are presented again at the same luminance (L2) as in the third step.

If the patient does not see some of the targets in the fourth step, a fifth step (205) is performed, in which only the luminance targets (M) not seen in the fourth step appear progressively, one by one, on the internal display (10) and the patient reports their vision with the patient interface (2). In the fifth step, the targets (M) have a third luminance (L3), e.g. 2.23 lux - 3.5db, which is higher than the second luminance (L2).

If the patient does not see some of the targets in the fifth step, a sixth step (206) is performed, in which the targets not seen in the fifth step are presented again at the same luminance (L3) as in the fifth step.

Evidently, the test can be repeated at the same luminance more than twice, or the test can be performed in more than six steps with targets with a progressively increasing luminance.

In each case, the test ends with a macular function coefficient (FM), the value of which is processed by an algorithm that performs the following formula:

$$FM = \frac{A1*C1 + A2*C2 + A3*C3}{B}$$

wherein

A1 are the targets seen in the first step and in the second step

A2 are the targets seen in the third step and in the fourth step

A3 are the targets seen in the fifth step and in the sixth step.

C1 is the ponderal coefficient assigned to the first step and to the second step

C2 is the ponderal coefficient assigned to the third step and to the fourth step

C3 is the ponderal factor assigned to the fifth step and to the sixth step

$$B = \frac{C1*A1}{100}$$ is a scaling factor to obtain a percentage result, giving the maximum percentage of 100 % to the case in which the patient sees all the targets in the first step and in the second step.

In the case where 17 luminance targets are presented in the first step, the following weights can be assigned to the ponderal coefficients:

C1 = 3
C2 = 2
C3 = 1

Therefore, in this case, the formula of the algorithm is given below

$$FM = \frac{A1*3 + A2*3 + A3*1}{0,51}$$

With reference to Fig. 8, during the test, a graph (31) appears on the external display (30) with colored points (P) in correspondence with all the luminance targets (M) that appeared in the internal display (10). In the graph (31), the grid (G) and the central fixation circle (C) are visible.

The colored points (P) are visible to the ophthalmologist during the test, and are characterized by different colors according to the test result.

Green-colored points correspond to the luminance targets seed by the patient during the first step and the second step; yellow-colored points correspond to the luminance targets seen by the patient in the third step and in the fourth step; red-colored points correspond to luminance targets seen by the patient in the fifth step and in the sixth step and finally black-colored points correspond to luminance targets not seen by the patient at the end of the test.

The graph (31) will be updated throughout the test point by point. At the end of the test, the external display (30) will show the final graph of the targets seen at the various steps and a value of the macular function coefficient (FM) expressed numerically as a percentage.

The graph (31) shows the test performed on the left eye. In this test the patient saw 12 targets out of 17 in the first step and in the second step, the patient saw 2 targets out of 5 in the third step and in the fourth step, and the patient saw 2 targets out of 3 in the fifth step and in the sixth step.

Therefore, the macular function coefficient will be:

$$FM = \frac{12*3 + 2*2 + 2*1}{0,51} = 82\%.$$

Only one target is unseen.
There are 2 false positives because the patient reported the luminance target outside the reaction time (t2) twice.

Fig. 8 illustrates a second graph (32) of a test performed on the right eye.

In this test the patient saw 11 out of 17 targets in the first step and in the second step, the patient saw 2 out of 6 targets in the third step and in the fourth step, and the patient saw 1 out of 4 targets in the fifth step and in the sixth step.

Therefore, the macular function coefficient will be:

$$FM = \frac{11*3 + 2*2 + 1*1}{0,51} = 74\%.$$

Three targets are unseen.
Variations and equivalent changes may be made to the present embodiment of the invention, within the scope of an expert of the field, which nevertheless fall within the scope of the invention expressed by the appended claims.

**Claims**

1. System (100) for macular function assessment comprising:

   - a visor (1) comprising an internal display (10),
   - a patient interface (2),
   - a user interface (3) comprising an external display (30),
   - a power supply (5), and
   - a control unit (4) operatively connected to the visor (1), to the patient interface (2), to the user interface (3), and to the power supply (5),
   wherein said control unit (4) is configured to display a background (S) and luminance targets (M) in the form of luminous points in the internal display (10) of the visor, said luminance targets (M) appearing one at a time at given locations in the background;
   wherein said patient interface (2) is configured to be operated by the patient to select each luminance target seen by the patient on the internal display (10);
   wherein said control unit (4) is configured to perform a test comprising a plurality of steps:

      wherein, in a first step (201), the N luminance targets (M) appear progressively, one by one, on the internal display (10) at a first luminance value (L1) and the patient reports the vision with the patient interface (2);
      wherein, in the steps after the first step (201), only the luminance targets not seen in the previous step appear progressively, one by one, on the internal display (10) at the same luminance value as the previous step or at a higher luminance value than the previous step and the patient reports the vision with the patient interface (2); wherein a macular function coefficient (FM) is calculated by means of an algorithm that takes into account the targets seen in the various steps of the test; and wherein the external display (30) of the user interface is configured to display a result of the test performed.

2. The system (100) according to claim 1, wherein the test comprises:

- a second step (202), wherein only the luminance targets (M) not seen in the first step appear progressively, one by one, on the internal display (10), at the first luminance value (L1) of the first step and the patient reports the vision with the patient interface (2);
- a third step (203), wherein only the luminance targets (M) not seen in the second step appear progressively, one by one, on the internal display (10), at a second luminance value (L2) that is higher than the first luminance value (L1) and the patient reports the vision with the patient interface (2); and
- a fourth step (204), wherein only the luminance targets (M) not seen in the third step appear progressively, one by one, on the internal display (10), at the second luminance value (L2) of the third step and the patient reports the vision with the patient interface (2).

3. The system (100) according to claim 2, wherein the test comprises:

- a fifth step (205), wherein only the luminance targets (M) not seen in the fourth step appear progressively, one by one, on the internal display (10), at a third luminance value (L3) higher than the second luminance value (L1) and the patient reports the vision with the patient interface (2); and
- a sixth step (206), wherein only the luminance targets (M) not seen in the fifth step appear progressively, one by one, on the internal display (10), at the third luminance value (L3) of the third step and the patient reports the vision with the patient interface (2).

4. The system (100) according to any one of the preceding claims, wherein said macular function coefficient (FM) is based on a number of luminance targets (N1, N2, N3) seen in each step and on ponderal coefficients (C1, C2, C3) assigned to each step.

5. The system (100) according to any one of the preceding claims, wherein said patient interface (2) comprises a joystick and/or buttons (K1, K2).

6. The system (100) according to any one of the preceding claims, wherein said external display (30) of the user interface (3) is a touch screen display.

7. The system (100) according to any one of the preceding claims, wherein the luminance targets (M) are located in an area defined by a grid (G) wherein the distances relative to a central fixation point (O) are measured in degrees; the extent of the grid (G) corresponding to the entire macular function area and being 12° vertically (6° in the upper quadrants and 6° in the lower quadrants relative to the central fixation point (O)) and 12° horizontally (6° in the nasal quadrant and 6° in the temporal quadrant relative to the central fixation point (O)).

8. The system (100) according to claim 7, wherein each luminance target (M) has a diameter of 0.6°.

9. The system (100) according to claim 7 or 8, wherein a circle (C) is drawn on the grid (G) defining a central fixation zone; said circle (C) having a center at the central fixation point (O) and a radius of 4°.

10. The system (100) according to claim 9, wherein the test provides for using 17 luminance targets, wherein nine luminance targets are located according to a 3x3 array inside the central fixation circle (C), and wherein one central luminance target is located at the central fixation point (O) and the other luminance targets are spaced by 2°, and eight luminance targets are located outside the central fixation circle (C) along eight equidistant points of a circumference with radius 6°.

11. The system (100) according to any one of the preceding claims, wherein said control unit (4) is configured to display a graph (31) with colored points (P) at all the luminance targets (M) that have appeared in the internal display (10) during the test on the external display (30) of the user interface; wherein said colored points are visible to the examiner during the test, and are **characterized by** different colors according to the result of the test.

12. The system (100) according to claim 11, when dependent on claim 10, wherein the grid (G) and the central fixation circle (C) are visible in the graph (31) of the external display (30).

FIG. 1

FIG. 2

FIG. 3

FIG. 4

FIG. 5

201 — FIRST STEP
FIRST LUMINANCE
VALUE L1

SEE ALL — YES → TEST END

NO

202 — SECOND STEP
SECOND LUMINANCE
VALUE L1

SEE ALL — YES → STEP END

$$FM = \frac{A1 \cdot C1}{B} = 100\%$$

203 — THIRD STEP
SECOND LUMINANCE
VALUE L2

SEE ALL — YES → FINE TEST

NO

204 — FOURTH STEP
SECOND LUMINANCE
VALUE L2

$$FM = \frac{A1 \cdot C1 + A2 \cdot C2}{B}$$

SEE ALL — YES → FINE TEST

NO

TO FIG. 7

FIG. 6

10

FROM FIG. 6

NO

205

FIFTH STEP
THIRD LUMINANCE
VALUE L3

SEE ALL — YES → TEST END

206

NO

SIXTH STEP
THIRD LUMINANCE
VALUE L3 → TEST END

$$FM = \frac{A1 \cdot C1 + A2 \cdot C2 + A3 \cdot C3}{B}$$

FIG. 7

**Duration**

02:17

**Macular Function
Left Eye** 82%

**Macular Function
Right Eye** DX 74%

SX          DX

Total Pts.      : 17/17
Seen Pts.       :    16
Not Seen Pts. :     1
False Positive:     2

TEST END

FIG. 8

Europäisches
Patentamt

European
Patent Office

Office européen
des brevets

# EUROPEAN SEARCH REPORT

**Application Number**

EP 25 17 1577

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/150727 A1 (BLAHA JAMES J [US] ET AL) 23 May 2019 (2019-05-23)<br>* paragraph [0046] *<br>* paragraph [0050] - paragraph [0052] *<br>* paragraph [0055] - paragraph [0066] *<br>* paragraph [0072] - paragraph [0074] *<br>* paragraph [0136] *<br>* paragraph [0141] - paragraph [0142] *<br>* paragraph [0267] *<br>* figures 2A-B, 6A-C, 19 *<br>----- | 1-12 | INV.<br>A61B3/024 |

TECHNICAL FIELDS
SEARCHED       (IPC)

A61B

The present search report has been drawn up for all claims

| Place of search | Date of completion of the search | Examiner |
|---|---|---|
| Munich | 1 August 2025 | Horváth, László |

EPO FORM 1503 03.82 (P04C01)

## ANNEX TO THE EUROPEAN SEARCH REPORT
## ON EUROPEAN PATENT APPLICATION NO.

EP 25 17 1577

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2025

| Patent document cited in search report | Publication date | Patent family member(s) | | Publication date |
|---|---|---|---|---|
| US 2019150727 A1 | 23-05-2019 | AU | 2018367510 A1 | 25-06-2020 |
| | | CA | 3082778 A1 | 23-05-2019 |
| | | CN | 111587086 A | 25-08-2020 |
| | | EP | 3709861 A1 | 23-09-2020 |
| | | JP | 2021502881 A | 04-02-2021 |
| | | JP | 2024009889 A | 23-01-2024 |
| | | US | 2019150727 A1 | 23-05-2019 |
| | | US | 2024099575 A1 | 28-03-2024 |
| | | WO | 2019099572 A1 | 23-05-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019150727 A1